# EUROPEAN PATENT SPECIFICATION

(11) **EP 1 562 449 B1**
(45) Date of publication and mention of the grant of the patent: **09.03.2011**
(21) Application number: 03779239.7
(22) Date of filing: 23.10.2003
(51) Int. Cl.: A23L 1/0522, A23L 1/302, A23L 2/00, A23L 2/38

(54) **COMPOSITION FOR ENHANCING PHYSICAL PERFORMANCE**
ZUSAMMENSETZUNG ZUR ERHÖHUNG DER LEISTUNGSFÄHIGKEIT DES KÖRPERS
COMPOSITION POUR AMELIORER LA PERFORMANCE PHYSIQUE

(30) Priority: 23.10.2002 US 420986 P; 22.11.2002 US 302544
(43) Date of publication of application: 17.08.2005
(73) Proprietor: The FRS Company, Lexington, MA 02421 (US)
(72) Inventor: LINES, Thomas Christian, L-5762 Hassel,Luxembourg Gran Duchy (LU); ONO, Mitsunori, Lexington, MA 02421 (US)
(74) Representative: Brookes Batchellor LLP
(86) International application number: PCT/US2003/033821
(87) International publication number: WO 2004/037018

(56) References cited:
- WO-A-00/12085
- WO-A-02/07768
- US-A1- 2002 025 350
- US-A1- 2003 068 391
- US-B1- 6 210 701
- US-B1- 6 261 589
- US-B1- 6 277 427
- US-B1- 6 299 925
- KOO MARCEL W L ET AL: "Pharmacological effects of green tea on the gastrointestinal system" EUROPEAN JOURNAL OF PHARMACOLOGY, vol. 500, no. 1-3, 1 October 2004 (2004-10-01), pages 177-185, XP004587635 ISSN: 0014-2999
- CHOW H -H SHERRY ET AL: "Phase I pharmacokinetic study of tea polyphenols following single-dose administration of epigallocatechin gallate and Polyphenon E" CANCER EPIDEMIOLOGY BIOMARKERS AND PREVENTION, vol. 10, no. 1, January 2001 (2001-01), pages 53-58, XP002366662 ISSN: 1055-9965

## Description

### BACKGROUND

Achieving peak physical performance has long been a goal for athletic competition and self-improvement. Means for improving physical performance includes prolonged systematic exercise, proper diet, and use of pharmaceuticals such as anabolic steroids. Anabolic steroids, which are testosterone derivatives, promote tissue growth, increase muscle mass, increase blood volume and hemoglobin level, and improve overall strength. Nonetheless, the use of anabolic steroids often results in serious complications, such as decreased blood high-density lipoprotein levels, disorders of the reproductive system, and disorders of the liver including carcinoma and peliosis hepatis. These complications further lead to virilization in females, interrupted growth in children, and defects in fetuses. The use of anabolic steroid can also cause psychological disorders such as unpredictable mood changes and aggression. Thus, there is a need for a safe drug or dietary supplement for enhancing physical performance.

### SUMMARY

This invention relates to a composition that contains quercetin and a number of other natural products. The composition is used in enhancing physical performance, i.e., improving an ability to perform an exercise, such as speed, strength, power, endurance, flexibility, agility, balance, focus coordination, reaction time, and fatigue recovery.

One aspect of this invention features a composition that contains quercetin and vitamin B3 and vitamin C. In one embodiment, the composition further contains at least one of the following ingredients: vitamin B1, vitamin B2, vitamin B6, and vitamin B12. In another embodiment, it further contains at least one of caffeine, epigallocatechin gallate, epicatechin, epicatechin gallate, epigallocatechin, and polypheron B. This composition may also contain other ingredients, such as vitamin E, CoQ-10, soy isoflavones, taurine, sugar beet pectin fiber, and a ginko biloba extract. Further, the composition can be sweetened, if necessary, by adding a sweetener, e.g., sorbitol, maltitol, cane sugar, high fructose corn syrup, and the like. The composition can also contain amino acids, minerals, a flavor enhancer, or a coloring agent. It is known that the leaves of green tea contain epigallocatechin gallate, epicatechin, epicatechin gallate, epigallocatechin, and polypheron E. Thus, these five ingredients can be conveniently provided as a green tea extract.

The composition of the invention can be in dry form (e.g., powder or tablet) or in aqueous form (e,g., beverage or syrup). It can be a dietary supplement or a pharmaceutical formulation. It can also be a drink or a food product. Examples include tea (e.g., a tea drink and the contents of a tea bag), soft drinks, juice (e.g., a fruit extract and a juice drink), milk, coffee, jelly, ice cream, yogurt, cookies, cereals, chocolates, and snack bars. The composition, in any of the forms described above, can be used to enhance physical performance. Also within the scope of this invention is a composition of the invention as an active agent.

The details of one or more embodiments of the invention are set forth in the accompanying description below. Other features, objects, and advantages of the invention will be apparent from the description and from the claims.

### DETAILED DESCRIPTION

This invention is based, at least in part, on the unexpected discovery that quercetin, an antioxidant, and a number of other natural products exhibit synergistic health benefits, including enhancing physical performance in a subject.

Within the scope of this invention is a quercetin-containing composition that includes vitamin B3 and vitamin C. It further contains one or more of vitamin B1, vitamin B2, vitamin B6, and vitamin B12. The composition can also contain one or more of caffeine, epigallocatechin gallate, epicatechin, epicatechin gallate, epigallocatechin, and polypheron E. A green tea extract can be conveniently used to provide epigallocatechin gallate, epicatechin, epicatechin gallate, epigallocatechin, and polypheron E.

Exemplary quantities of the ingredients of this composition are: 0.1-50 mg of vitamin B1, 0.1-150 mg of vitamin B2, 0.1-2000 mg of vitamin B3, 0.1-200 mg of vitamin B6, 5-150 µg of vitamin B12, 50-2000 mg of vitamin C, 50-1500 mg of caffeine, 20-2000 mg of quercetin, 10-500 mg of epigallocatechin gallate, 10-500 mg of epicatechin, 10-500 mg of epicatechin gallate, 10-500 mg of epigallocatechin, and 10-500 mg of polypheron E, which can be dissolved or dispersed in a 1 L aqueous solution. The quantities of the ingredients can also be those of the same relative ratio to those listed above. The term "quercetin" refers to both quercetin aglycon and quercetin derivatives, e.g., quercetin-3-O-glucoside, quercetin-5-O-glucoside, quercetin-7-O-glucoside, quercetin-9-O-glucoside, quercetin-3-O-rutinoside, quercetin-3-O-[α-rhamnosyl-(1→2)-α-rhamnosyl-(1→6)]-β-glucoside, quercetin-3-O-galactoside, quercetin-7-O-galactoside, quercetin-3-O-rhamnoside, and quercetin-7-O-galactoside. After digestion, quercetin derivatives are converted to quercetin aglycon, an active form absorbed in the body. The quantity of quercetin mentioned above refers to that of quercetin aglycon or the quercetin moiety of a quercetin derivative. As an example, a composition for daily use can be a 1 L aqueous solution containing 1000 mg of quercetin, 30 mg of vitamin B1, 85 mg of vitamin B2, 1 g of vitamin B3,100 mg of vitamin B6,120 µg of vitamin B12,1200 mg of vitamin C, 1000 IU of vitamin E, 1000 mg of caffeine, and a green tea extract containing 120 mg of epigallocatechin gallate, 140 mg of epicatechin, 360 mg of epicatechin gallate, 360 mg of epigallocatechin, and 120 mg of polypheron E.

This composition may also contain one or more other active ingredients, such as vitamin E, CoQ-10, soy isoflavones, taurine, sugar beet pectin fiber, and a ginko biloba extract. Exemplary quantities of these ingredients are: 3-1000 IU of vitamin E, 10-400 mg of CoQ-10, 20-600 mg of soy isoflavones, 10-1000 mg of taurine, 1-15 g of sugar beet pectin fiber, and 50-500 mg of a ginko biloba extract (dry weight). Further, the composition can be sweetened, if necessary, by adding a sweetener such as sorbitol, maltitol, hydrogenated glucose syrup and hydrogenated starch hydrolyzate, high fructose corn syrup, cane sugar, beet sugar, pectin, and sucralose.

An example of the above-described composition is a powder. It can be used conveniently to prepare beverages, e.g., tea or juice. The powder can also be used to prepare paste, jelly, capsules, or tablets. Lactose and corn starch are commonly used as diluents for capsules and as carriers for tablets. Lubricating agents, such as magnesium stearate, are typically added to form tablets.

The composition of this invention can also be a dietary supplement or a pharmaceutical formulation. As a dietary supplement, additional nutrients, such as minerals or amino acids may be included. The composition can also be a drink or food product. As used herein, the terms "drink" and "food" broadly refer to any kinds of liquid and solid/semi-solid materials, respectively, that are used for nourishing an animal, and for sustaining normal or accelerated growth of an animal including a human. Examples of the drink product include, but are not limited to, tea-based beverages, juice, coffee, and milk. Examples of the food product include jelly, cookies, cereals, chocolates, snack bars, herbal extracts, dairy products (e.g., ice cream, and yogurt), soy bean product (e.g., tofu), and rice products.

The above-described composition, in any of the forms described above, can be used for enhancing physical performance. As shown in the examples below, the composition improves overall strength, balance, fatigue recovery, intensity of physical exercise, and endurance to the exercise.

The terms "improving", "treating," and "lowering" refer to the administration of an effective amount of a composition of the invention to a subject, who needs to improve his physical performance, with the purpose to improve physical performance. The term "administration" covers oral or parenteral delivery to a subject a composition of the invention in any suitable form, e.g., food product, beverage, tablet, capsule, suspension, and solution. The term "parenteral" refers to subcutaneous, intracutaneous, intravenous, intramuscular, intraarticular, intraarterial, intrasynovial, intrasternal, intrathecal, intralesional, and intracranial injection, as well as various infusion techniques. An "effective amount" refers to a dose of the composition that is sufficient to provide a physical benefit (e.g., improving endurance).

Both *in vivo* and *in vitro* studies can be conducted to determine optimal administration routes and doses. The specific examples below are to be construed as merely illustrative, and not limitative of the remainder of the disclosure in any way whatsoever. Without further elaboration, it is believed that one skilled in the art can, based on the description herein, utilize the present invention to its fullest extent.

### Example 1

Composition A (1000 ml) was prepared by mixing the following ingredients at room temperature: 1000 ml of orange juice, 1000 mg of quercetin, 30 mg of vitamin B1, 85 mg of vitamin B2, 1000 mg of vitamin B3, 100 mg of vitamin B6,120 µg of vitamin B 12, 1000 IU of vitamin E, and 1000 mg of caffeine. All ingredients were obtained from Spectrum Laboratory Products, Inc., Gardena, CA; Sigma, St. Louis, MO; and Aldrich, Milwaukee, WI.

Ten male Spregue-Dawley rats, weighing 240-250 g, were obtained from Charles River Lab (Boston, MA). The rats were divided into Groups 1 and 2 (5 in each group). The rats in the Group 2 were administered by intragastric feeding with the just-described composition at a daily dose of 8 ml/rat (30 ml/kg body weight) for 48 days. The rats in Group 1 were administered with water.

At days 0, 14, 28, and 42 after the administration, blood samples were collected from the rats by supraorbital bleeding and various hematological parameters
were determined using standard methods. The results are summarized in Tables 1 and 2 below.

**Table 1. Effects of composition A on rat hematological parameters**

| | | **Day 0** | | **Day14** | | **Day 28** | | **Day 42** | |
|---|---|---|---|---|---|---|---|---|---|
| **Parameter** | **Reference Range** | **Group 1** | **Group 2** | **Group 1** | **Group 2** | **Group 1** | **Group 2** | **Group 1** | **Group 2** |
| WBC | 9.4-14.9 (THSN/UL) | 17.22 | 15.04 | 17.34 | 16.50 | 18.76 | 16.90 | 17.66 | 14.20 |
| RBC | 6.2-9.0 (MILL/UL) | 6.09 | 5.94 | 6.63 | 6.60 | 7.43 | 7.20 | 7.99 | 7.72 |
| Hb | 13.4-1G.4 (GM/DL) | 12.46 | 12.38 | 14.24 | 14.40 | 15.32 | 15.02 | 15.8G | 15.18 |
| Hematocrit | 40.0-49.0 (%) | 37.80 | 37.44 | 42.30 | 43.26 | 45.56 | 45.00 | 47.04 | 45.86 |
| MCV | 52.0-66.0 (FL) | 62.20 | 63.20 | 63.80 | G5.40 | G1.60 | 62.60 | 58.80 | 59.40 |
| MCH | 17.7-19.1 (PICO GM) | 20.46 | 20.84 | 21.50 | 21.82 | 20.68 | 20.86 | 19.88 | 19.72 |
| MCHC | 32.0-33.5 (%) | 32.96 | 33.00 | 33.64 | 33.26 | 33.66 | 33.38 | 33.72 | 33.14 |
| Platelet | 780-1400 (THSN/UL) | 956.00 | 965.00 | 1084.60 | 1158.80 | 1078.40 | 1076.60 | 967.00 | 962.80 |
| BANDS | 0.00-0.06 (THSN/UL) | 0.00 | 0.00 | 0.00 | 0.00 | 0.00 | 0.00 | 0.00 | 0.00 |
| Segmented Neutrophiles | 0.58-6.30 (THSN/UL) | 4.44 | 4.78 | 3.34 | 3.59 | 3.41 | 3.33 | 3.27 | 3.60 |
| Lymphocyte | 3.78-14.9 (THSN/UL) | 10.07 | 7.84 | 12.10 | 11.02 | 13.52 | 11.99 | 12.78 | 9.37 |
| Monocyte | 0.02-1.20 (THSN/UL) | 3.43 | 2.15 | 1.67 | 1.67 | 1.49 | 1.30 | 1.24 | 1.03 |
| Eosinophiles | 0.00-0.01(THSN/UL) | 0.54 | 0.16 | 0.11 | 0.10 | 0.17 | 0.13 | 0.20 | 0.10 |
| Basophiles | 0.00-0.00(THSN/UL) | 0.14 | 0.11 | 0.12 | 0.12 | 0.17 | 0.15 | 0.16 | 0.09 |
| Atipicle Lymphocyte | 0.00-0.00 (THSN/UL) | 0.02 | 0.00 | 0.00 | 0.00 | 0.00 | 0.00 | 0.00 | 0.00 |
| Metamyelocytes | 0.00-0.00 (THSN/UL) | 0.00 | 0.00 | 0.00 | 0.00 | 0.00 | 0.00 | 0.00 | 0.00 |
| Myelocytes | 0.00-0.00 (THSN/UL) | 0.00 | 0.00 | 0.00 | 0.00 | 0.00 | 0.00 | 0.00 | 0.00 |
| NRBC/100WBC | 0-0 (/IOOWBC) | 0.60 | 0.40 | 0.00 | 0.00 | 0.00 | 0.00 | 0.00 | 0.00 |
| **Reticulocyte** | **0.1-4.0(%)** | **1** | **1** | **2.62** | **5.74** | **3.30** | **5.90** | **4.78** | **6.58** |

| | | | | | | | | | |
|---|---|---|---|---|---|---|---|---|---|
| Note: WBC: white blood cell RBC: red blood cell Hb: hemoglobin MCV: mean corpuscular volume MCH: hemoglobin amount per red blood cell MCHC: mean cell hemoglobin concentration BANDS: premature neutrophil NRBC: nucleated red blood cell count THSN/UL: 1,000/ul MILL/UL: 1,000,000/ul GM/DL: gram/dl FL: femtoliter PICO GM: picogram | | | | | | | | | |

**Table 2. Effects of composition A on rat reticulocyte level**

| **Group** | **Ref. Range (%)** | **Day** | **Reticulocyte level in each rat** | | | | | **Average** | **SD** |
|---|---|---|---|---|---|---|---|---|---|
| | | | 1# | 2# | 3# | 4# | 5# | | |
| 1 | 0.1-4.0 | 14 | 2.9 | 2.9 | 2 | 4.9 | 4.4 | 2.62 | 0.96 |
| 2 | 0.1-4.0 | 14 | 5.8 | 4.9 | 7.5 | 5.8 | 4.7 | 5.74 | 2.15 |
| 1 | 0.1-4.0 | 28 | 3.1 | 2.2 | 2.2 | 7.2 | 6.8 | 3.30 | 2.5 |
| 2 | 0.1-4.0 | 28 | 4.8 | 6.5 | 5.4 | 5.5 | 73 | 5.90 | 1.3 |
| | | | | | | | | | |
| 1 | 0.1-4.0 | 42 | 4.8 | 9.2 | 9 | 5.5 | 5.4 | 4.78 | 2.14 |
| 2 | 0.1-4.0 | 42 | 7.5 | 6.2 | 6.1 | 6.4 | 6.7 | 6.58 | 0.16 |

| | | | | | | | | | |
|---|---|---|---|---|---|---|---|---|---|
| Note: SD = Standard Deviation | | | | | | | | | |

As shown in Tables 1 and 2, the reticulocyte levels in the rats administered with composition B (Group 2) were higher than those in the rats administered with water (Group 1). For example, at Day 42, the average reticulocyte level in the rats of Group 2 (6.58%) was higher than that in the rats of Group 1 (4.78%) by 37.7%. On the other hand, other hematological parameters of the rats in the two groups did not differ significantly See Table 1. These results indicate that composition A increases the reticulocyte level but does not affect other hematological parameters. Reticulocytes are immature, anucleated red blood cells (RBCs). An increase in the reticulocyte level and no changes in other hematological parameters suggest that composition A improves the renewal of RBC.

During the experiment, the body weight of each rat was monitored daily. No statistical difference was found between the two groups.

### Example 2

Composition B (1000 ml) was prepared by mixing the following ingredients at room temperature: 1000 ml of orange juice, 1000 mg of quercetin, 30 mg of vitamin B1, 85 mg of vitamin B2, 1000 mg of vitamin B3, 100 mg of vitamin B6, 120 µg of vitamin B12, 1000 IU of vitamin E, 1000 mg of caffeine, 500 mg of epigallocatechin gallate, 500 mg of epicatechin, 500 mg of epicateqin gallate, 500 mg of epigallocatechin, and 500 mg of polypheron E.

Ten male Spregue-Dawley rats that weighed 240-250 g were divided into Groups 1 and 2 (5 in each group). The rats in Group 2 were administered by intragastric feeding with composition B at an average daily dose of 14 ml/kg body weight for 95 days. Those in Group 1 were administered with water.

Starting from Day 92 after the administration, each of the rats was trained on a Rota-Rod treadmill (Model 57750, Stoelting Co., Wood Dale, Illinois) for over 2 hours. At Day 95, after being trained for another 20 minutes, each of the rats was put on the treadmill and allowed to walk. The time for which each rat walked on the treadmill before falling off was recorded and the average time for the rats in Groups 1 and 2 determined. The experiments were repeated for three times ("Test A," "Test B," and "Test C"). The results are summarized in Table 3 below.

**Table 3. Effects of composition B**

| Groups | Time on Rota-Rod treadmill (min) | | |
|---|---|---|---|
| Group 1 | Test A | TestB | TestC |
| #1 | 2.36 | 13.11 | 23.33 |
| #2 | 10.69 | 16.02 | 44.21 |
| #3 | 19.02 | 15.46 | 66.90 |
| #4 | 2.99 | 16.67 | 16.09 |
| #5 | 1.34 | 3.41 | 7.82 |
| Average | **7.28** | **12.93** | **31.67** |
| SE | 3.37 | 2.45 | 10.68 |
| Group 2 | | | |
| #1 | 6.54 | 61.95 | 80.40 |
| #2 | 16.16 | 21.54 | 41.73 |
| #3 | 6.91 | 23.83 | 90.47 |
| #4 | 24.19 | 20.42 | 202.82 |
| #5 | 32.58 | 15.37 | 67.44 |
| Average | **17.28** | **28.62** | **96.57** |
| SE | 5.03 | 8.45 | 27.79 |

| | | | |
|---|---|---|---|
| Note: SE = Standard Error | | | |

As shown in Table 3, the rats got used to the exercise and walked for longer time on the treadmill as the experiment went on. In all tests, the rats that had been administered with composition B walked on the treadmill longer than those that had been not. These results indicate that composition B enhanced the physical performance of rats. During the 95 days of administration, the body weight of each rat was monitored daily. No statistical difference was found between Groups 1 and 2. This result suggests the enhanced physical performance of the rats in Group 2 was not due to an increase in body mass.

## Claims

1. Non therapeutic use of a composition comprising vitamin B3, quercetin, and vitamin C in improving overall strength, fatigue recovery, and endurance to exercise in a mammal.

2. The use of claim 1, the composition further comprising at least one of vitamin B1, vitamin B2, vitamin B6, vitamin B12, and vitamin E.

3. The use of claim 1 or claim 2, the composition further comprising at least one of caffeine, epigallocatechin gallate, epicatechin, epicatechin gallate, epigallocatechin, and polypheron E.

4. The use of any one of claims 1-3, the composition further comprising a green tea extract containing at least one of caffeine, epigallocatechin gallate, epicatechin, epicatechin gallate, epigallocatechin, and polypheron E.

5. The use of any one of claims 1-4, the composition further comprising at least one of CoQ-10, soy isoflavones, sugar beet pectin fiber, and a ginko biloba extract.

6. The use of any one of claims 1-5, the composition further comprising 20-2000 mg quercetin, 0.1-2000 mg vitamin B3, and 50-2000 mg vitamin C, or in quantities of the same ratio.

7. The use of any one of claims 1-5, the composition comprising an aqueous solution.

8. The use of any one of claims 1-6, wherein the composition is in dry form.

9. The use of any one of claims 1-7, wherein the composition is tea, soft drinks, juice, milk, coffee, jelly, ice cream, yogurt, cookies, cereals, chocolates, or snack bars.

10. The use of any one on claims 1 -9, the composition further comprising taurine.

11. The use of claim 10, wherein the taurine is present in an amount of 10-1000 mg.

## Patentansprüche

1. Nichttherapeutische Verwendung einer Zusammensetzung, die Vitamin B3, Quercetin und Vitamin C umfasst, beim Verbessern der Gesamtstärke, Ermüdungserholung und Übungsausdauer in einem Säuger.

2. Die Verwendung gemäß Anspruch 1, wobei die Zusammensetzung ferner wenigstens einen der folgenden Stoffe umfasst: Vitamin B1, Vitamin B2, Vitamin B6, Vitamin B12 und Vitamin E.

3. Die Verwendung gemäß Anspruch 1 oder Anspruch 2, wobei die Zusammensetzung ferner wenigstens einen der folgenden Stoffe umfasst:
Koffein, Epigallocatechingallat, Epicatechin, Epicatechingallat, Epigallocatechin und Polypheron E.

4. Die Verwendung gemäß einem der Ansprüche 1 bis 3, wobei die Zusammensetzung ferner Grünteeextrakt umfasst, umfassend wenigstens einen der folgenden Stoffe: Koffein, Epigallocatechingallat, Epicatechin, Epicatechingallat, Epigallocatechin und Polypheron E.

5. Die Verwendung gemäß einem der Ansprüche 1 bis 4, wobei die Zusammensetzung ferner wenigsten einen der folgenden Stoffe umfasst:
CoQ-10, Soja-Isoflavone, Zuckerrübenpektinfaser und ein Ginkgo-Bilobaextrakt.

6. Die Verwendung gemäß einem der Ansprüche 1 bis 5, wobei die Zusammensetzung ferner 20 bis 2000 mg Quercetin, 0,1 bis 2000 mg Vitamin B3 und 50 bis 2000 mg Vitamin C oder Mengen derselben Verhältnisse umfasst.

7. Die Verwendung gemäß einem der Ansprüche 1 bis 5, wobei die Zusammensetzung eine wässrige Lösung umfasst.

8. Die Verwendung gemäß einem der Ansprüche 1 bis 6, wobei die Zusammensetzung in der Trockenform vorliegt.

9. Die Verwendung gemäß einem der Ansprüche 1 bis 7, wobei die Zusammensetzung in Form von Tee, Softdrinks, Saft, Milch, Kaffee, Gelee, Eiscreme, Joghurt, Keksen, Müsli, Schokoladen oder Müsliriegel vorliegt.

10. Die Verwendung gemäß einem der Ansprüche 1 bis 9, wobei die Zusammensetzung ferner Taurin umfasst.

11. Die Verwendung gemäß Anspruch 10, wobei das Taurin in einer Menge von 10 bis 1000 mg vorliegt.

## Revendications

1. Utilisation non thérapeutique d'une composition comprenant de la vitamine B3, de la quercétine, et de la vitamine C, pour améliorer la résistance globale, la récupération de la fatigue, et l'endurance à un exercice chez un mammifère.

2. Utilisation selon la revendication 1, la composition comprenant en outre au moins un composé parmi la vitamine B1, la vitamine B2, la vitamine B6, la vitamine B12, et la vitamine E.

3. Utilisation selon la revendication 1 ou la revendication 2, la composition comprenant en outre au moins un composé parmi la caféine, le gallate d'épigallocatéchine, l'épicatéchine, le gallate d'épicatéchine, l'épigallocatéchine et le polyphéron E.

4. Utilisation selon l'une quelconque des revendications 1 à 3, la composition comprenant en outre un extrait de thé vert comprenant au moins un composé parmi la caféine, le gallate d'épigallocatéchine, l'épicatéchine, le gallate d'épicatéchine, l'épigallocatéchine et le polyphéron E.

5. Utilisation selon l'une quelconque des revendications 1 à 4, la composition comprenant en outre au moins un composé parmi le CoQ-10, les isoflavones de soja, la fibre de pectine de betterave sucrière, et un extrait de gingko biloba.

6. Utilisation selon l'une quelconque des revendications 1 à 5, la composition comprenant en outre de 20 à 2000 mg de quercétine, de 0,1 à 2000 mg de vitamine B3, et de 50 à 2000 mg de vitamine C, ou d'autres quantités selon le même rapport.

7. Utilisation selon l'une quelconque des revendications 1 à 5, la composition comprenant une solution aqueuse.

8. Utilisation selon l'une quelconque des revendications 1 à 6, où la composition se présente sous une forme sèche.

9. Utilisation selon l'une quelconque des revendications 1 à 7, où la composition est du thé, des boissons sans alcool, du jus, du lait, du café, de la gelée, de la glace, du yaourt, des cookies, des céréales, des chocolats ou des barres de collation.

10. Utilisation selon l'une quelconque des revendications 1 à 9, la composition comprenant en outre de la taurine.

11. Utilisation selon la revendication 10, où la taurine est présente suivant une quantité comprise entre 10 et 1000 mg.
